# EUROPEAN PATENT SPECIFICATION

(11) **EP 2 630 961 B1**
(45) Date of publication and mention of the grant of the patent: **26.11.2014**
(21) Application number: 13168694.1
(22) Date of filing: 22.10.2007
(51) Int. Cl.: A61K 35/74, A61P 17/02, C12N 1/16

(54) **Agent for promoting healing of a wound of a living body**
Mittel zur Förderung der Wundheilung eines lebenden Körpers
Agent favorisant la guérison d'une blessure d'un organisme vivant

(30) Priority: 02.11.2006 JP 2006298478
(43) Date of publication of application: 28.08.2013
(62) Divisional of application: 07830257.7
(73) Proprietor: Aureo Co., Ltd., Tokyo 108-0071 (JP)
(72) Inventor: Moriya, Naoyuki, Tokyo, Tokyo 1080071 (JP); Moriya, Yukiko, Tokyo, 1080071 (JP); Kubota, Koji, Tokyo, Tokyo 1080071 (JP)
(74) Representative: Beacham, Annabel Rose

(56) References cited:
- EP-A1- 1 602 377
- WO-A1-2006/032530
- D. PIKAZIS ET AL: "Extended fungal skin infection due to Aureobasidium pullulans", CLINICAL AND EXPERIMENTAL DERMATOLOGY, vol. 34, no. 8, 1 December 2009 (2009-12-01), pages e892-e894, XP55066176, ISSN: 0307-6938, DOI: 10.1111/j.1365-2230.2009.03663.x

## Description

### BACKGROUND OF THE INVENTION

### 1. Field of the Invention

The present invention relates to a living body healing accelerator containing as an active constituent a mixture of a culture solution obtained after cultivation of a microorganism belonging to the *Aureobasidium sp.* and mycelia of the microorganism cultivated in the culture solution.

### 2. Description of the Related Art

A culture solution obtained after cultivation of a microorganism belonging to the *Aureobasidium sp.* is now used as a food or beverage product or as a food additive. The culture solution contains β-glucan, and it is known that the β-glucan is 1,3,1,6-β-D-glucan having a glucose residue with β-1,3 linkage as a principal chain and a glucose residue with β-1,6 linkage coupled thereto as a side chain (or a branched chain). (Acta Chemica Scandinavia 17, 1351-1356 (1963) and Agric. Biol. Chem. 47(6), 1167-1172 (1983))

JP 2002-204687 A discloses the fact that a culture solution obtained after cultivation of a microorganism belonging to the *Aureobasidium sp.* shows high anti-tumor activity and high immunostimulation activity when orally administered and can be used as a preventive drug or a curative drug to various types of diseases.

However, it has not been reported yet that the mixture of the culture solution obtained after cultivation of a microorganism belonging to the *Aureobasidium sp.* and mycelia of the microorganism cultivated in the culture solution exhibits an effect on cells and tissues for enhancing their regeneration ability.

JP 2002-204687 A also discloses the fact that the culture solution obtained after cultivation of a microorganism belonging to the *Aureobasidium sp.* can be used as a preventive or curative drug for various types of diseases, and lists diabetes as an example of the diseases. However, JP 2002-204687 A does not disclose any testal data suggesting that the culture solution is active in prevention or therapeutic effect of diabetes.

JP 2002-204687 A also describes that the culture solution obtained after cultivation of a microorganism belonging to the *Aureobasidium sp.* is effective in suppression of proliferation of leukemia cells in an test system using cultured cells, but does not describe any testal data indicating the pharmaceutical effect of the culture solution based on a result of tests with animals.

EP1602377 discloses compositions comprising *Aureobasidium pullulans* M1 for use as a skin moistening agent.

Dissection of skin or mucosa performed in association with a surgical operation and injuries caused by burning or laceration can be regarded as damage to cells or tissues. When dissection is performed, various preventive treatments are performed, such as prevention of infectious diseases caused by bacteria by oral administration of an antibiotic or direct application of the antibiotic drug to an external wound, reduction of inflammation by administration of an anti-inflammatory drug, or reduction of pain by administration of an analgetic.

The medical treatment for a wound of skin or mucosa caused by a surgical operation aims only reduction of secondary disturbances such as infection of bacteria, inflammation, and pain. Currently healing of the wound is dependent on skin regeneration ability of the living body's. Because of such circumstances, a long time is required until recovery, and there are still concerns about the possibility of side effects caused by administration of drugs.

To solve the problem described above, active efforts have been made for development of a drug capable of accelerating the ability of healing that a living body originally has, but there is still not any drug that can sufficiently satisfy all of the requirements in the effect, safety, production cost, and the like.

As described above, there is the strong need for providing a living body healing accelerator capable of accelerating healing of damages to tissues caused by a wound such as a burn in the clinical field.

### SUMMARY OF THE INVENTION

An object is to provide, for solving the problems so ever, a living body healing accelerator which is capable of accelerating regeneration ability of a living body, leading to healing of damages of cells or tissues caused by a wound, having high safety, and also not causing any side effect even administered for a long period of time.

To solve the problems described above, the inventors of the present invention devotes themselves to find out natural materials originated from organisms which has high safety to a human body and are present in abundance in nature as natural resources. During the research process, the inventors found that (1) a mixture of a culture solution obtained after cultivation of a microorganism belonging to the *Aureobasidium sp*. and mycelia of the microorganism cultivated in the culture solution shows an accelerating activity in the regeneration ability relating to the cure of damages of cells or tissues caused by a wound in a living body. Based on the facts described above, the inventors found that the mixture is useful as a living body healing accelerator, and completed the present invention.

According to an embodiment of the present invention, provided is a living body healing accelerator including as an active constituent a mixture of a culture solution obtained after cultivation of a microorganism belonging to the *Aureobasidium* pullulans and mycelia of the microorganism cultivated in the culture solution.

According to another embodiment of the present invention, provided is a use of a mixture of a culture solution obtained after cultivation of a microorganism belonging to the *Aureobasidium* pullulans and mycelia of the microorganism cultivated in the culture solution in manufacturing a medicament for for topical use in therapy in accelerating healing of a wound.

The living body healing accelerator is used for acceleration of healing of a wound. In this case, the wound is caused in association with a surgical operation or by burn on skin, and the living body healing accelerator is preferably directly applied to an affected area.

Further, in the present invention, the microorganism belonging to the *Aureobasidium* pullulans is preferably the *Aureobasidium pullulans* strain M-1 (Deposited number of FERM BP-08615 at International Patent Organism Depositary, National Institute of Advanced Industrial Science and Technology (Independent Administrative Corporation)) or the *Aureobasidium pullulans* strain M-2 (Deposited number of FERM BP-10014 at International Patent Organism Depositary, National Institute of Advanced Industrial Science and Technology (Independent Administrative Corporation)).

With the present invention, it is possible to provide a living body healing accelerator containing as an active constituent a mixture of a culture solution obtained after cultivation of a microorganism belonging to the *Aureobasidium* pullulans and mycelia of the microorganism cultivated in the culture solution, and acceleration in healing of a wound such as a burn can be performed by applying the living body healing accelerator to the wound.

As described above, with the living body healing accelerator according to the present invention, it is possible to accelerate activity in the regeneration ability relating to healing of damages of skin and tissues in a living body.

### BRIEF DESCRIPTION OF THE DRAWINGS

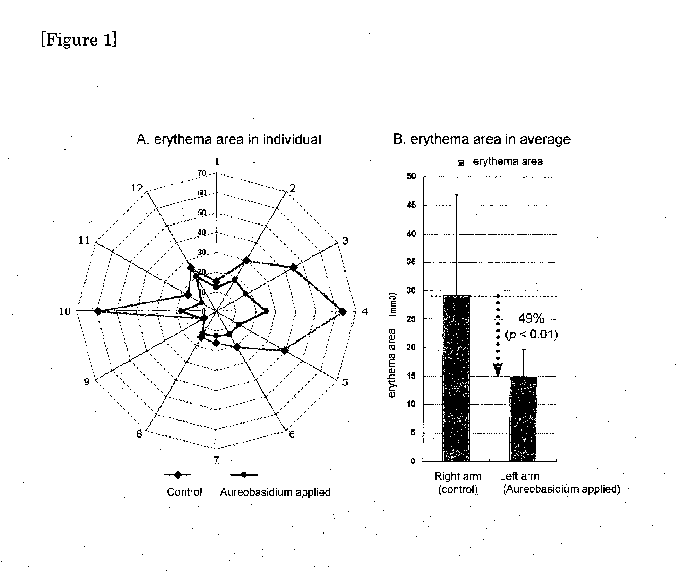

Fig. 1 is a view illustrating a result of comparison of erythema areas in 3 days after cautery treatment.

### DETAILED DESCRIPTION OF THE INVENTION

The present invention is described in detail below.

The term of "microorganism belonging to the *Aureobasidium* sp." as used herein is applicable to any strain separated from the environment (such as food products, soil, or indoor environment).

The most preferable example of the microorganism belonging to the *Aureobasidium sp.* is strains belonging to *Aureobasidium pullulans.* More specifically, the preferable microorganisms include strains stocked as single strains respectively such as IFO 4464, IFO 4466, IFO 6353, IFO 7757, ATCC 9348, ATCC 3092, ATCC 42023, and ATCC 433023. Especially, the *Aureobasidium pullulans* strain M-1 (Deposited number of FERM BP-08615 at International Patent Organism Depositary, National Institute of Advanced Industrial Science and Technology (Independent Administrative Corporation)) is the most preferable one because of the high yield of 1,3,1,6-β-D-glucan.

It is also possible to use a mutated strain, which is prepared by subjecting a stock strain separated as a single strain to the ordinary technology for mutation. The ordinary technology for mutation as used herein include, but not limited to UV radiation, or chemical processing with such chemicals as nitrosoguanidine, ethidium bromide, ethyl methanesulfonate, or sodium nitrite, and especially the *Aureobasidium pullulans* strain M-2 (Deposited number of FERM BP-10014 at International Patent Organism Depositary, National Institute of Advanced Industrial Science and Technology (Independent Administrative Corporation)) is the most preferable one because of the high yield of 1,3,1,6-β-D-glucan, and also because accumulation of color in a culture solution is lower as compared to the parent strain M-1.

"A mixture of a culture solution obtained after cultivation of a microorganism belonging to the *Aureobasidium* sp. and mycelia of the microorganism cultivated in the culture solution" according to the present invention is obtained by culturing microorganisms belonging to the *Aureobasidium sp..* The microorganism belonging to the *Aureobasidium sp.* can be cultured according to any of known methods (Refer to, for instance, Japanese Patent Laid-open Publication No. Sho 57-149301). That is, the culture may require inoculating bacteria in a culture medium (with the pH of 5.2 to 6.0) containing a 0.5 to 1.0 mass% of carbon source (such as sucrose), 0.1 mass% of a nitrogen source, and extremely small amounts of other substances (such as vitamins, or inorganic materials), and cultivating the bacteria for 2 to 3 days at a temperature in the range from 20 to 30 °C in the aerated state, preferably in the aerated state with agitation. When β-1,3-1,6-gulcan produces, viscosity of the culture solution becomes higher, and a gel-like material having high viscosity is generated. The culture solution obtained as described above generally contains 0.6 to 1.8 mass% of a solid phase, and 5 to 80 mass% of the β-1,3-1,6-gulcan is contained in the solid phase. In the present invention, it is preferable to use a culture containing 1 mass% or more of β-1,3-1,6-gulcan of the solid phase, and is more preferable to use a culture containing 5 mass% or more of β-1,3-1,6-gulcan in the solid phase. When a concentration of β-1,3-1,6-gulcan in the culture is too low, the sufficient physiological effect of the glucan can not be expected.

Quantification of β-1,3-1,6-gulcan can be performed according to the method described in Japanese Examined Patent Publication No. Hei 3-48201. That is, after culturing is finished, the culture solution is sterilized and is subjected to centrifugation to remove the fungus bodies. Then a mixture solution of chloroform and butanol is added in the obtained solution by 10 volume % and the resultant mixture solution is shaked (sevage method) and then to centrifugation to remove chloroform and insoluble matters. The operating sequence is repeated twice, and then the solution is subjected to ethanol precipitation to recover precipitated materials. The recovered materials are dissolved in distilled water, and pullulan is decomposed by processing with an enzyme. Then the decomposed materials are subjected to dialysis in distilled water, and the dialysis solution is subjected to ethanol precipitation to recover the precipitated material (β-1,3-1,6-gulcan) and determine the yield.

The culture solution obtained as described above may be used after sterilized by heating or pressurizing, or may be used after condensed or dried according to the necessity. Culture of bacteria belonging to *Aureobasidium sp.* are used as food additives like a thickening stabilizer, and the safety is high.

When the mixture of the culture solution obtained after cultivation of the microorganism belonging to the *Aureobasidium sp.* and mycelia of the microorganism cultivated in the culture solution is condensed, the condensation is preferably performed at low temperature in the depressurized state. The condensation may be performed until the mixture is dried and solidified. In this case, it is more preferably to perform freeze drying or spray drying in the depressurized state. It is to be noted that the mixture may be filtered before condensation and then condensed.

The "mixture of the culture solution obtained after cultivation of the microorganism belonging to the *Aureobasidium sp.* and mycelia of the microorganism cultivated in the culture solution" may be further purified. Chromatography or elution using an ion exchange resin may be employed singly or in combination for purifying the mixture.

When chromatography is employed, any of normal phase chromatography, reversed phase chromatography, high-performance liquid chromatography, centrifugal liquid chromatography, column chromatography, thin-layer chromatography and the like may be used singly or in combination. Conditions for purification, such as a carrier and a solvent for elution may be selected according to the necessity in correspondence to the selected chromatography. For instance, in a case of normal phase chromatography, a chloroform methanol-based solvent may be used, and in reversed phase chromatography, a water-methanol-based solvent may be used.

As the elution method using an ion exchange resin, for instance, the method may be employed in which the eluted liquid is diluted and dissolved in water or lower alcohol, the solution is contacted to the ion exchange resin to be adsorbed therein and then eluted with water or lower alcohol. The lower alcohol used in this method is as described above, and methanol is especially preferable. There is no specific restriction over the ion exchange resin to be used in this method on the condition that the ion exchange resin can be used for purification in this technical field, and for instance, any of a porous and cross-linked polystyrene-based resin having a large mesh-like structure, urbanlite, cellulose, and the like may be used.

The "living body healing accelerator" is used for accelerating regeneration of cells and tissues damaged by a wound. The uses include, for instance, acceleration of healing of a tissue damaged by a wound caused by dissection of skin or mucosa performed in association with a surgical operation, burn, laceration, or the like.

The "living body healing accelerator" may be orally administered, and also may be administered as an external preparation which is applied to skin or the like. When the drug is used for accelerating healing of a tissue damaged by a wound caused by dissection of skin or mucosa performed in association with a surgical operation, burn, or laceration, the living body healing accelerator is preferably directly applied to the affected area.

The "living body healing accelerator" may be sold as it is as a product in the market, but generally various types of constituents for improving the taste or for being formed into a required form are added and blended therein, and further, a flavor is added to prepare a final product.

The constituents added and blended in the "living body healing accelerator" include various types of sugars, emulsifying agents, sweeteners, acidifiers, fruit juices or the like. More specifically, sugars such as glucose, sucrose, fructose, honey; sugar alcohols such as sorbitol, xylitol, erythritol, lactitol, and palatinit; and emulsifying agents such as sucrose fatty acid ester, glycerin sugar fatty acid ester, and lecithin may be used as the constituents. In addition, various types of vitamins such as vitamin A, vitamin B, vitamin C, and vitamin E, or extracts of herb, constituents of cereals, constituents of vegetables, and constituents of milk may be added and blended therein to obtain living body healing accelerator having an excellent taste.

The flavors which may be added to the "living body healing accelerator" include those based on yogurt, berries, oranges, Chinese quince, Japanese basil, citrus, apple, mint, grape, pear, custard cream, peach, melon, banana, tropical, herb, black tea, coffee, and the like, and the flavors may be used singly or in combination. There is no specific restriction over an amount of the added flavor, and the amount of added flavor is preferably in the range from 0.05 to 0.5 mass%, and more preferably in the range from about 0.1 to about 0.3 mass% for providing a good taste.

The "living body healing accelerator" described above can be processed into market products in any states including a solid state, a liquid state, and the like.

The "living body healing accelerator" can be used as various forms of preparations such as granules, tablets, or capsules prepared by any known technology in the field of drug and food production, together with medicinal acceptable salts, diluents, preserving agents, coloring agents, and taste-adjusting agents.

Further, the "living body healing accelerator", can be used for health food. The health food means foods especially aimed for maintaining and promoting welfare and health more positively as compared to ordinary foods. More specifically, the foods include, liquid, semisolid or solid products, such as cookie, rice cracker, jelly, sweet jelly of beans, yogurt, steamed bread, other types of cakes, cold beverage, energy drink, soup, and the like. In addition, the "living body healing accelerator" may be decocted to be a tea form. Further, the living body healing accelerator can be processed into health foods by being added, by means of mixing, applying, or spraying, during the final stage of the production process or into the final products.

A dosage of the "living body healing accelerator" varies according to age, symptom, and other factors of a recipient. For instance, when orally administered, one dosage for an adult is 3 to about 100 g when used in the gel-like state, and 200 mg to about 3 g when used in the powder state. When used as an extract, the dosage is in the range from 50 mg to about 2 g according to a level of purification or a moisture content, and in any case, the accelerator is preferably administered 3 times a day and about 30 minutes before each meal. When used as a health food, an amount of the material to be used does not affect a taste or an appearance of the food, and for instance, when used in the powder state, the dosage is preferably in the range from 1 to about 100 g against 1 kg of the food.

Further, the "living body healing accelerator" can be used as an external preparation for skin in various product forms such as lotion (cosmetic water), creams or the like for cosmetics, emulsions, cosmetic water, pack drugs, skin milk (emulsion), gel, powder, lip creams, lip rouge, under makeup, foundation, sunblock creams, bath agents, body shampoos, body rinse agents, soap, cleansing foam, ointments, patches, jelly-like preparations, and aerosol agents.

When the "living body healing accelerator" is used as an external preparation for skin, various constituents or additives as described below, which are generally used in cosmetics, quasi drugs, and drugs, may be blended therein according to the necessity.

That is, the various constituents or additives which may be blended in the living body healing accelerator include, but not limited to, moisturizing agents such as glycerin, vaseline, urea, hyaluronic acid, heparin; ultraviolet absorbers or scattering agents such as PABA derivatives (para-aminobenzoic acid, Escalora 507, or the like), cinnamic acid derivatives (such as neoheliopan, parsol MCX, and sunguard B), salicylic acid derivatives (such as octyl salicylate), benzophenone derivatives (such as ASL-24, ASL-24S), dibenzoylmethane derivatives (such as Parsol A, Parsol DAM), heterocyclic derivatives (tinuvin-based one or the like), titanium oxide; metal chelators such as disodium edetate, trisodium edetate, citric acid, sodium citrate, tartaric acid, sodium tartrate, lactic acid, malic acid, sodium polyphosphate, sodium methaphosphate, and gluconic acid; sebum-preventing agents such as salicylic acid, sulfur, caffeine, tannin; germicides or disinfectants such as benzarconium chlorate, benzethonium chlorate, and chlorhexidine gluconate; anti-inflammatory agents such as diphenhydramine chlorate, tranexamic acid, guaiazulene, azulene, allantoin, hinokitol, glycyrrhizic acid and salts thereof, glycyrrhizic acid derivatives, and glycyrrhetenic acid; vitamins such as vitamin A, vitamin B group (B1, B2, B6, B12, and B15), folic acid, nicotine acids, pantothenic acids, biotin, vitamin C, vitamin D group (D2, D3), vitamin E, ubiquinones, vitamin K (K1, K2, K3, and K4); amino acids and derivatives thereof, such as asparagine acid, glutamic acid, alanine, lysine, glycine, glutamine, serine, cysteine, cystine, tyrosine, proline, arginine, and pyrrolidone carbonate; whitening agents such as retinol, tocopherol acetate, magnesium ascorbyl-phosphate, ascorbic acid glucoside, arbutin, kojic acid, ellagic acid, and placental extract fluids; antioxidants such as butylhydroxytoluene, butylhydroxyanisole, and propyl gallate; astringent drugs such as zinc chloride, zinc sulfate, zinc phenol, zinc oxide, and aluminate-potassium sulfate; sugars such as glucose, fructose, maltose, sucrose, trehalose, erythritol, mannitol, xylitol, and lactitol; and essences from various plants such as licorice root, matricaria, horse chestnut, strawberry geranium, paeoniae radix, Chinese quince, Scutellaria root, cork tree bark, coptis rhizome, Houttuyniae Herba, and maidenhair tree. In addition, oily substances, surfactants, thickifiers, alcohols, powder substances, coloring agents, and the like may be added in the living body healing accelerator depending on the necessity.

When the "living body healing accelerator" is used as an external preparation for skin, the application quantity is preferably in the range from 10 to about 1,000 mg/cm² in a case of a gel and in the range from 1 to about 50 mg/cm² in a case of powder. In a case of the extract, the quantity is preferably in the range from 1 to 500 mg/m² according to a degree of purification, a moisture content, or the like.

### Examples

The present invention is described below in further detail with reference to examples thereof, but it is to be noted that the present invention is not restricted by the examples in any sense.

In the tests described below, a mixture of a culture solution prepared by culturing the *Aureobasidium pullulans* strain M-2 according to the known method and mycelia therein (with the solid phase contents of 1.5 to 2 mass%; hereinafter referred to as Aureobasidium culture solution) was used.

### (Example 1) (Test for acceleration of healing of burn)

In the test, burn was prepared by burning moxa at specific regions called Kyokuchi of both arms of 12 test subjects respectively (age of each test subject is as shown in Table 2), the culture solution of *Aureobasidium sp.* was applied to the burn on the left arm to compare influence of the effects over healing of the burn with that on the right arm which was not subjected to any specific treatment.

More specifically, moxa was burnt on the regions called Kyokuchi on both arms of each test subject to prepare burn, and then a patch of absorbent cotton having the size of 1.5 cm x 1.2 cm with 1.5 mL of Aureobasidium culture solution absorbed therein was adhered to the site of the left arm where moxa was burnt and fixed with adhesive tapes, and the absorbent cotton patch was removed when the cotton was dried (in 4 to 5 hours). The treatment was performed once a day for three days. On the other hand, not specific treatment was carried out to the burn on the right arm. A erythema caused by the burn was photographed every day, and subjective symptoms concerning pain, burning feeling, blister, and itch were inquired to each test subject.

**[Table 1]**

| Background of test subjects | | |
|---|---|---|
| Item | Classification | Number of Examples (%) |
| Gender | Male | 8 (66.7) |
| | Female | 4 (33.3) |
| Age | 30 to less than 40 | 3 (25.0) |
| | 40 to less than 50 | 5 (41.7) |
| | 50 to less than 60 | 2 (16.7) |
| | 60 to less than 70 | 1 (8.3) |
| | 70 to less than 80 | 1 (8.3) |

A result in 3 days after application of moxa is as shown in FIG. 1. FIG. 1A shows values of erythema areas on each test subject, while FIG. 1B shows the average values. A remarkable difference is observed on each of the test subjects No. 3, No. 4, No. 5, and No. 10, and it was confirmed that, on the left arm of each test subject to which the Aureobasidium culture solution was applied, the erythema area was reduced to 51% of that on the right arm not subjected to any specific treatment with the statistically significant difference (p<0.01).

This result suggest that healing of the burn was accelerated by application of the Aureobasidium culture solution. The conceivable mechanism seems to be attributed to acceleration of regeneration of cells and tissues, and the effect emerges as accelerated regeneration of a tissue damaged due to acceleration in healing of a burn.

Based on the results as described above, it could be confirmed that the Aureobasidium culture solution accelerates healing of a burn.

### Industrial Applicability

It was confirmed that, by using the Aureobasidium culture solution, healing of a wound caused by burn can be accelerated.

It may be said that acceleration of healing of tissues damaged, for instance, by a burn is very useful for maintaining social activities of the burnt person.

## Claims

1. A living body healing accelerator comprising as an active constituent a mixture of a culture solution obtained after cultivation of an *Aureobasidium pullulans* and mycelia of the microorganism cultivated in the culture solution, for topical use in therapy in accelerating healing of a wound.

2. A living body healing accelerator according to claim 1, wherein the wound is caused in association with a surgical operation or by a burn on skin, and the living body healing accelerator is directly applied to an affected area.

3. A living body healing accelerator according to claim 1 or claim 2, wherein the *Aureobasidium pullulans* is the *Aureobasidium pullulans* strain M-1 deposited with number FERM BP-08615 at the International Patent Organism Depositary, National Institute of Advanced Industrial Science and Technology (Independent Administrative Corporation) or the *Aureobasidium pullulans* strain M-2 deposited with number FERM BP-10014 at the International Patent Organism Depositary, National Institute of Advanced Industrial Science and Technology (Independent Administrative Corporation).

4. Use of a mixture of a culture solution obtained after cultivation of an *Aureobasidium pullulans* and mycelia of the microorganism cultivated in the culture solution in manufacturing a medicament for topical use in therapy in acceleration of healing a living body, wherein the medicament is for accelerating healing of a wound.

5. A use according to claim 4, wherein the wound is caused in association with a surgical operation or by burn on skin, and the medicament is directly applied to an affected area.

6. Use according to any one of claims 4 or 5, wherein the *Aureobasidium pullulans is* the *Aureobasidium pullulans* strain M-1 deposited with number FERM BP-08615 at the International Patent Organism Depositary, National Institute of Advanced Industrial Science and Technology (Independent Administrative Corporation) or the *Aureobasidium pullulans* strain M-2 deposited with number FERM BP-10014 at the International Patent Organism Depositary, National Institute of Advanced Industrial Science and Technology (Independent Administrative Corporation).

## Patentansprüche

1. Heilungsbeschleuniger für einen lebenden Körper, umfassend als wirksamen Bestandteil eine Mischung einer Kulturlösung, die nach der Kultivierung von *Aureobasidium pollulans* und Myzelien des Mikroorganismus, der in der Kulturlösung kultiviert wird, erhalten wird, zur topischen Verwendung in der Therapie für die Beschleunigung der Heilung einer Wunde.

2. Heilungsbeschleuniger für einen lebenden Körper nach Anspruch 1, wobei die Wunde im Zusammenhang mit einem chirurgischen Eingriff oder einer Verbrennung der Haut verursacht wird und der Heilungsbeschleuniger für einen lebenden Körper direkt auf eine betroffene Fläche aufgetragen wird.

3. Heilungsbeschleuniger für einen lebenden Körper nach Anspruch 1 oder Anspruch 2, wobei *Aureobasidium pullulans* der *Aureobasidium pullulans*-Stamm M-1 ist, hinterlegt mit der Nummer FERM BP-08615 beim International Patent Organism Depositary, National Institute of Advanced Industrial Science and Technology (Selbstverwaltungskörperschaft) oder der *Aureobasidium pullulans*-Stamm M-2 ist, hinterlegt mit der Nummer FERM BP-10014 beim International Patent Organism Depositary, National Institute of Advanced Industrial Science and Technology (Selbstverwaltungskörperschaft).

4. Verwendung einer Mischung einer Kulturlösung, die nach Kultivierung von *Aureobasidium pollulans* und Myzelien von dem Mikroorganismus, der in der Kulturlösung kultiviert wurde, erhalten wird, zur Herstellung eines Medikaments für die topische Verwendung in der Therapie zur Beschleunigung der Heilung eines lebenden Körpers, wobei das Medikament zum Beschleunigen der Heilung einer Wunde ist.

5. Verwendung nach Anspruch 4, wobei die Wunde im Zusammenhang mit einem chirurgischen Eingriff oder einer Verbrennung der Haut verursacht wird und das Medikament direkt auf eine betroffene Fläche aufgetragen wird.

6. Verwendung nach einem der Ansprüche 4 oder 5, wobei *Aureobasidium pullulans* der *Aureobasidium pullulans-*Stamm M-1 ist, hinterlegt mit der Nummer FERM BP-08615 beim International Patent Organism Depositary, National Institute of Advanced Industrial Science and Technology (Selbstverwaltungskörperschaft) oder der *Aureobasidium pullulans*-Stamm M-2 ist, hinterlegt mit der Nummer FERM BP-10014 beim International Patent Organism Depositary, National Institute of Advanced Industrial Science and Technology (Selbstverwaltungskörperschaft).

## Revendications

1. Un accélérateur de guérison d'un corps vivant comprenant comme constituant actif une mixture d'une solution de culture obtenue après culture d'un *Aureobasidium pullulans* et de mycéliums du micro-organisme cultivés dans la solution de culture, pour utilisation topique en thérapie en accélérant la guérison d'une plaie.

2. Un accélérateur de guérison d'un corps vivant selon la revendication 1, dans lequel la plaie est causée en association avec une opération chirurgicale ou par une brûlure de la peau, et l'accélérateur de guérison d'un corps vivant est appliqué directement sur une zone affectée.

3. Un accélérateur de guérison d'un corps vivant selon la revendication 1 ou la revendication 2, dans lequel l'*Aureobasidium pullulans* est la souche *Aureobasidium pullulans* M-1 déposée sous le numéro FERM BP-08615 à l'Organisme de Dépôt International des Brevets, Institut National pour la Science et les Technologies Industrielles Avancées (Entreprise Administrative Indépendante) ou la souche *Aureobasidium pullulans* M-2 déposée sous le numéro FERM BP-10014 à l'Organisme de Dépôt International des Brevets, Institut National pour la Science et les Technologies Industrielles Avancées (Entreprise Administrative Indépendante).

4. Utilisation d'une mixture d'une solution de culture obtenue après culture d'un *Aureobasidium pullulans* et de mycéliums du microorganisme cultivés dans la solution de culture pour la fabrication d'un médicament pour utilisation topique en thérapie en accélération de guérison d'un corps vivant, dans laquelle le médicament est pour accélérer la guérison d'une plaie.

5. Une utilisation selon la revendication 4, dans laquelle la plaie est causée en association avec une opération chirurgicale ou par une brûlure de la peau, et le médicament est appliqué directement sur une zone affectée.

6. Utilisation selon une quelconque des revendications 4 ou 5, dans laquelle l'*Aureobasidium pullulans* est la souche *Aureobasidium pullulans* M-1 déposée sous le numéro FERM BP-08615 à l'Organisme de Dépôt International des Brevets, Institut National pour la Science et les Technologies Industrielles Avancées (Entreprise Administrative Indépendante) ou la souche *Aureobasidium pullulans* M-2 déposée sous le numéro FERM BP-10014 à l'Organisme de Dépôt International des Brevets, Institut National pour la Science et les Technologies Industrielles Avancées (Entreprise Administrative Indépendante).
